# EUROPEAN PATENT SPECIFICATION

(11) **EP 2 083 902 B1**
(45) Date of publication and mention of the grant of the patent: **30.08.2017**
(21) Application number: 07827228.3
(22) Date of filing: 18.10.2007
(51) Int. Cl.: A61F 2/01

(54) **FILTER ASSEMBLIES**
FILTERANORDNUNGEN
ENSEMBLES FILTRE

(30) Priority: 18.10.2006 US 852392 P; 18.10.2006 US 582354
(43) Date of publication of application: 05.08.2009
(73) Proprietor: Inspiremd Ltd., 67448 Tel-aviv (IL)
(72) Inventor: HOLZER, Asher, 34406 Haifa (IL); BAR, Eli, 30875 Moshav Megadim (IL); PAZ, Ofir, 75238 Rishon-lezion (IL)
(74) Representative: Abraham, Richard
(86) International application number: PCT/IL2007/001254
(87) International publication number: WO 2008/047368

(56) References cited:
- WO-A1-01/30266
- WO-A1-88/09683
- WO-A1-99/65417
- WO-A1-2006/116636
- WO-A2-03/022325
- WO-A2-2007/067451
- US-B2- 6 340 364

## Description

This Application claims benefit of priority from U.S. Patent Application No. 11/582,354 and U.S. Provisional Application No. 60/852,392, both filed 18 October 2006.

### FIELD AND BACKGROUND OF THE INVENTION

The present invention, in some embodiments thereof, relates to vascular filters that filter debris from the blood. More particularly, the present invention relates to vascular filters that expand in conjunction with radially expandable vascular flow-increasing devices.

In 1977 Andreas Gruntzig performed the first successful balloon angioplasty on an obstructed human artery, thereby opening the vessel and allowing improved flow of blood. While providing a tremendous advance in cardiology, angioplasty could not prevent restenosis, wherein tissue at the angioplasty site again became blocked.

The use of stents to prevent restenosis in treated stenotic vasculature began in 1994 following U.S. Food and Drug Administration approval of the Palmaz-Schatz stent.

A problem associated with balloon angioplasty and stent deployment is that during radial expansion of the radially expandable vascular flow-increasing device against the stenotic lesion, the stenotic lesion may release debris that travels to vital organs, for example the brain and/or lungs, causing vascular blockage, tissue necrosis and/or patient death.

To prevent such draconian sequela, a number of *in vivo* debris filter assemblies have been developed that are designed to be deployed distal to a radially expandable vascular flow-increasing device and capture debris released from stenotic lesions. A distal filter typically comprises a porous flexible material supported by a stiff frame.

Prior to introducing the stent and/or balloon, a distal filter is expanded at a site distal to the stenotic lesion. The balloon and/or stent is then guided into place proximate to the stenotic lesion and expanded. As blood passes through the filter, debris generated by the radial outward expansion of the balloon and/or stent is captured in the filter.

The filter is collapsed at the end of the procedure, trapping the debris.

In balloon angioplasty, the filter is pulled out of the vasculature trailing the balloon. In stent deployment, the filter is pulled through the stent during retrieval. Examples of expandable vascular filters can be found in: U.S. Patent No. 6,391,044 (Yadav et al); U.S. Patent No. 5,814,064 (Daniel et al); U.S. Patent No. 4,723,549 (Wholey et al); and U.S. Patent No. 5,827,324 (Cassell et al).

WO 99/65417 discloses a filter assembly in accordance with the precharacterising portion of claim 1.

### SUMMARY OF THE INVENTION

Some embodiments of the present invention successfully address at least some of the shortcomings of the prior art by providing a filter that is operatively connected to a radially expandable vascular flow-increasing device.

In accordance with the present invention, there is provided a filter assembly as defined in appendant independent claim 1, to which reference should now be made.

In some embodiments, the radially expandable vascular flow-increasing device comprises: at least one balloon configured to volumetrically expand and, during at least a portion of the expansion, operatively connect with a filter, and to contract following the expansion, and the operative connection comprises an operative connection between the filter and the at least one balloon during at least a portion of the volumetric expansion of the at least one balloon.

In some embodiments, the at least one balloon comprises at least one proximal portion and at least one distal portion.

In some embodiments, the filter operatively connects with the balloon at least one of: the at least one proximal portion, and the at least one distal portion.

In some embodiments, a maximal expansion diameter of the at least one distal portion of the at least one balloon is greater than a maximal expansion diameter of the at least one proximal portion of the at least one balloon.

In some embodiments, a maximal expansion diameter of the at least one proximal portion of the at least one balloon is greater than a maximal expansion diameter of the at least one distal portion of the at least one balloon.

In some embodiments, the at least one balloon comprises at least one angioplasty balloon.

In some embodiments, at least a portion of the filter is configured to remain removably connected to a luminal aspect during the contraction of the at least one balloon.

In some embodiments, the assembly includes at least one cord operatively associated with the filter and configured to disconnect at least a portion of the filter from the luminal aspect when tension is applied to the at least one cord.

In some embodiments, at least a portion of the filter includes a pressure-sensitive adhesive having an affinity for a tissue associated with an *in vivo* luminal aspect.

In some embodiments, the adhesive is an adhesive from the group of adhesives comprising fibrin, biological glue, collagen, hydrogel, hydrocolloid, collagen alginate, and methylcellulose.

In some embodiments, at least a portion of the filter is configured to remain removably connected to the luminal aspect during the contraction of the at least one balloon.

In some embodiments, the assembly includes at least one cord operatively associated with the filter and configured to disconnect the at least a portion of the filter from the luminal aspect when tension is applied to the at least one cord.

In some embodiments, the assembly includes a compression sleeve comprising a substantially curved wall having a proximal end, a distal end and a lumen extending from the proximal end to the distal end, the lumen having a cross sectional diameter that is substantially smaller than the maximal cross sectional diameter of the luminal aspect.

In some embodiments, the assembly includes at least one cord operatively associated with the filter, at least a portion of the at least one cord movingly juxtaposed within the compression sleeve lumen.

In some embodiments, when the at least one cord operatively associated with the filter is held relatively stationary during a first distal moving of the compression sleeve, the filter is caused to disconnect from the luminal aspect.

In some embodiments, in response to at least one second distal moving of the sleeve while the at least one cord is held relatively stationary, the filter is caused to radially contract such that a maximal cross sectional diameter of the filter is smaller than a cross sectional diameter of the sleeve lumen.

In some embodiments, in response to at least one third distal moving of the sleeve while the at least one cord is held stationary, at least a portion of the filter is caused to enter the sleeve lumen.

In some embodiments, the at least one balloon comprises an outer wall having a distal end and a proximal end and an inner wall defining a lumen, the lumen extending from the distal end to the proximal end, and at least a portion of the at least one cord is configured to slidingly pass through the lumen.

In some embodiments, the at least one cord is configured to pull at least a portion of the filter into contact with the distal end of the at least one balloon.

In some embodiments, the filter includes a distal portion, a proximal portion, an opening to the filter associated with the proximal portion and at least one strut operatively associated with the proximal portion.

In some embodiments, the assembly includes at least one cord operatively associated with the at least one strut, such that at least a portion of the opening is configured to contract radially inwardly in response to tension applied to the at least one cord.

In some embodiments, the at least one strut comprises at least two struts, at least one first strut and at least one second strut, the at least two struts being operatively associated with the at least one cord.

In some embodiments, the at least two struts are configured to resiliently flex outward with respect to a longitudinal axis passing through a center of the filter during at least a portion of the volumetric expansion of the at least one balloon.

In some embodiments, during at least a portion of the outward flexion of the at least two struts: the at least one first strut forms at least one first radius, and the at least one second strut forms at least one second radius with respect to the longitudinal axis.

In some embodiments, the filter includes: a distal portion, a proximal portion, an opening to the filter associated with the proximal portion, and at least one cord guide channel circumferentially encircling at least a portion the proximal portion.

In some embodiments, the assembly includes at least one cord, at least a portion of the at least one cord passes through the guide channel, such that at least a portion of the opening is configured to contract radially inwardly in response to tension applied to the at least one cord.

In some embodiments, the radially expandable vascular flow-increasing device comprises: at least one balloon configured to volumetrically expand and, during at least a portion of the expansion, operatively connect with a filter, and, following the connection, to contract following the expansion, the filter comprises a material having tissue connective properties for a portion of luminal tissue associated with an *in vivo* fluid stream, and the operative connection comprises an operative connection between the filter and the at least one balloon during at least a portion of the volumetric expansion of the at least one balloon.

In some embodiments, the radially expandable vascular flow-increasing device comprises: a radially expandable stent configured to open a stenotic lumen, the radially expandable stent having a proximal end, a distal end and a lumen connecting the proximal and the distal ends, an expandable balloon mounted on a distal portion of an elongate catheter, the expandable balloon configured to expand within the lumen of the expandable stent and cause the expandable stent to expand, and the operative connection comprises an operative connection between the filter and the expandable stent.

In some embodiments, the filter comprises a billowing filter.

In some embodiments, the expandable opening of the filter is removably connected to the stent.

In some embodiments, the assembly includes at least one cord operatively associated with the filter and configured to disconnect at least a portion of the filter from the expandable stent when tension is applied to the at least one cord.

In some embodiments, the expandable opening of the filter is operatively connected with the stent such that expansion of the stent causes expansion of the expandable opening of the filter.

In some embodiments, the radially expandable vascular flow-increasing device comprises: a radially expandable stent configured to open a stenotic lumen, the radially expandable stent having a proximal end, a distal end and a lumen connecting the proximal and the distal ends, and the operative connection comprises an operative connection between the filter and the expandable stent.

In some embodiments, the expanding stent is self-expanding.

In some embodiments, the assembly includes a stent holding spindle operatively associated with the stent when the stent is in a contacted configuration, the spindle being mounted on a distal portion of an elongate catheter.

In some embodiments, the stent holding spindle includes a channel.

In some embodiments, the assembly includes at least one cord operatively associated with the filter, at least a portion of the at least one cord being configured to slidingly pass through the channel through the spindle.

In some embodiments, the at least one cord is configured to pull at least a portion of the filter opening into contact with at least a portion of the spindle.

In some embodiments, the stent comprises a self-expanding stent and the assembly includes a compression sleeve comprising a substantially curved wall having a proximal end, a distal end and a lumen extending from the proximal end to the distal end, the compression sleeve configured to slidingly encircle the stent when the stent is in a contracted configuration.

In some embodiments, the assembly includes at least one cord, a portion of the at least one cord being movingly juxtaposed within the compression sleeve lumen.

In some embodiments, the filter includes at least one cord guide channel circumferentially encircling at least a portion the proximal opening of the filter.

In some embodiments, the assembly includes at least one cord, at least a portion of the at least one cord passes through the guide channel, such that at least a portion of the opening is configured to contract radially inwardly in response to tension applied to the at least one cord.

In some embodiments, the assembly includes a belt that provides the operative connection between the jacket and the filter.

In some embodiments, the belt is looped in at least one loop that removable connects the expandable opening of the filter with the jacket.

In some embodiments, a tension applied to the belt causes the loop to disconnect from the expandable opening of the filter with the jacket, thereby removing the operative connection between the opening to the filter and the jacket.

In some embodiments, the belt includes at least one connector that removably connects the filter to the jacket, the at least one connector from the group comprising a hook and a zipper.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figures la-3e show stent and debris filter assemblies being deployed in vessels shown in cross section;
Figures 4a-4f show jacketed stents;
Figures 5a-6h show jacketed stent and debris filter assemblies being deployed in vessels shown in cross section, according to embodiments of the invention;
Figure 7a-7d show deployment of an *in vivo* filter and balloon assembly in a vessel shown in cross section; and
Figures 8a-8d, 9a-9c, 10, and 11a-11e show alternative arrangements to the filter and balloon assembly shown in Figures 7a-7d.

### DESCRIPTION OF THE PREFERRED EMBODIMENTS

The present invention relates to vascular filters that filter debris from the blood and, more particularly, to vascular filters that expand in conjunction with a radially expandable vascular flow- increasing device to filter debris from the blood.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Embodiments of the present invention relate to vascular filters that expand in conjunction with radially expandable vascular flow-increasing devices. The phrase "radially expandable vascular flow-increasing devices" refers to, *inter alia,* balloon catheters, balloon catheters in conjunction with stents, jacketed stents and self expanding stents.

### Balloon, Stent and Filter Assembly 100

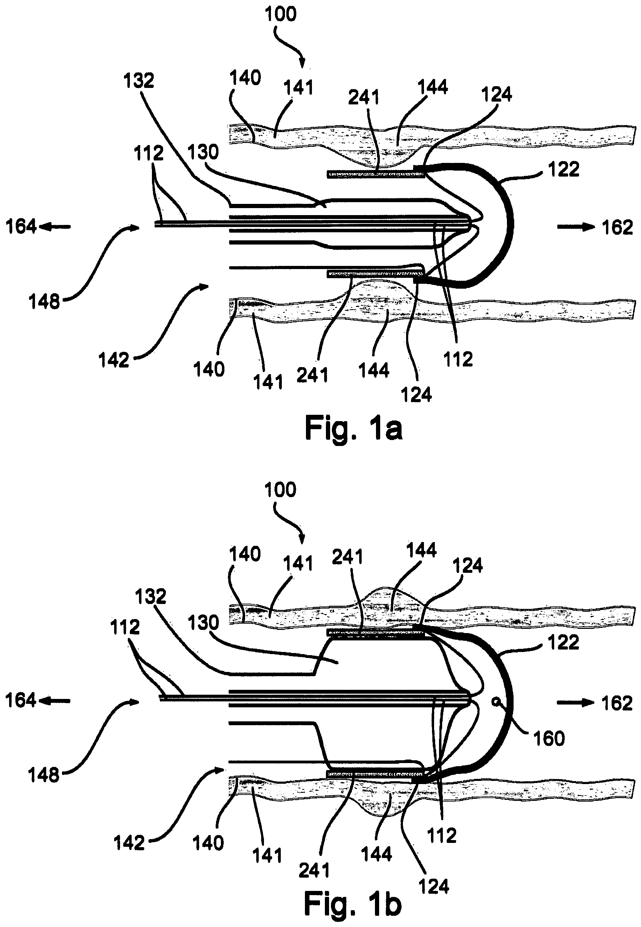

Figure 1a shows a representation of an *in vivo* stent filter assembly 100 in a cross section of a blood vessel 141; assembly 100 comprising a stent 241 and a filter 122.

In this example, stent 241 is positioned adjacent a stenotic lesion 144 with a catheter balloon. 130 inside stent 241. As seen in Figure Ib, balloon 130 is inflated to press stent 241 radially outward. Filter 122 has an opening 124 that is removably attached to stent 241 so that during expansion of stent 241, filter 122 is biased into an open position to span a blood vessel lumen 142.

Filter opening 124 is configured to flex radially outward until limited by a luminal aspect 140, having, for example, a diameter of between 3.0 and 6.0 millimeters, depending on the size of lumen 142 in which filter 122 is deployed. Filter 122 typically comprises a mesh sheet material that is configured to filter debris 160 from lumen 142.

As stenotic lesion 144 is cracked and squashed radially outwards by the expansion of stent 241, fluid passes through vessel lumen 142 in a distal or downstream direction 162 and carries debris 160 into filter 122. As used herein, the terms distal and distally refer to a position and a movement, respectively, in downstream direction 162.

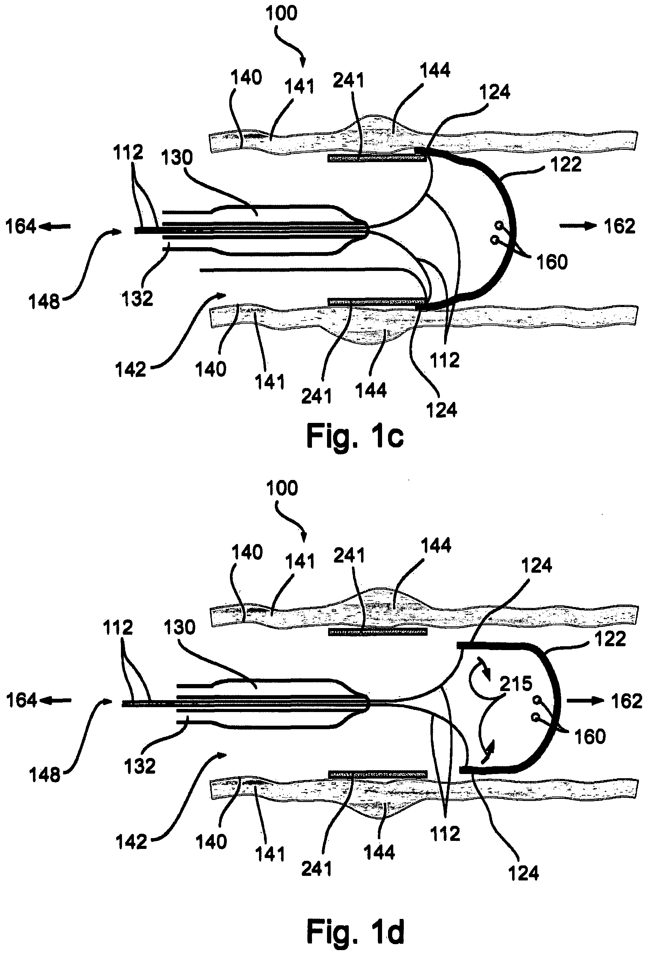

As seen in Figure 1c, after balloon 130 is deflated, filter 122 remains in position against luminal aspect 140 as a result of the radially expanded position of stent 241 against filter opening 124.

As seen in Figure 1d, cords 112 attached to filter opening 124, exit filter 122 and pass through a catheter channel 148 passing through balloon 130 and a catheter 132. When all debris 160 has been blocked by filter 122, cords 112 are pulled proximally by an *ex vivo* operator, in an upstream direction 164 to cause filter opening 124 to contract radially inward in a direction 215 and disconnect from stent 241. As used herein, the terms proximal and proximally refer to a position and a movement, respectively, in upstream direction 164.

In one example, filter 122 is configured to disconnect from luminal aspect 140 in response to tension applied to cords 112 of at least about one Newton and no more than about 20 Newtons.

As the diameter of lumen 142 is larger than the diameter of catheter channel 148, continued upstream pull on cords 112 proximally 164 biases filter opening 124 radially inward in direction 215. Fluid in lumen 142 travels distally in direction 162 so that pulling catheter 132 and filter 122 proximally 164 causes debris 160 to move downstream against filter 122. Debris 160 remains captured by filter 122 even as filter 122 moves with filter opening 124 in a fully or partially open position, as might occur when there is considerable volume of debris 160, for example in large arteries.

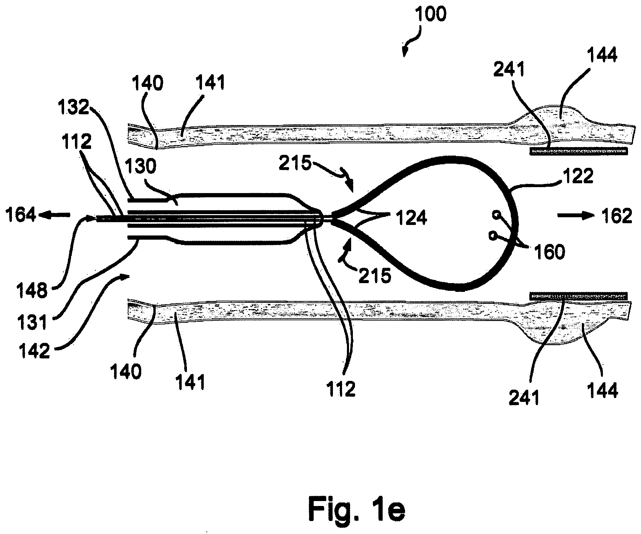

As seen in Figure 1e, continued pulling on cords 112 proximally 164 causes a portion of filter 122 to pass through stent 241 and contact balloon 130 and/or enter catheter channel 148. Filter 122 and catheter 132 are then pulled together proximally 164 percutaneously through lumen 142 until reaching the *ex vivo* environment.

Stent 241 typically includes a metallic base, for example stainless steel, nitinol, tantalum, MP35N alloy, a cobalt-based alloy, platinum, titanium, or other biocompatible metal alloys. During expansion of stent 241, filter opening 124 is substantially supported by stent 241, reducing the need for support by an integral stiff frame as is known in the art.

Such a filter 122, herein billowing filter 122, has substantially reduced bulk over filters in the art that have support frames. Billowing filter 122 will readily contract to an amorphous small mass that easily passes through stent 241 without spilling debris.

Billowing filter 122 extends directly from stent 241 and contacts unhealthy tissue of luminal aspect 140. Due to proximity to stent 241, billowing filter 122 is substantially unlikely to cause damage to healthy tissue of luminal aspect 140.

The absence of the above-noted support structure allows billowing filter opening 124 to gently contact luminal aspect 140. Billowing filter opening 124, therefore substantially prevents damage in special situations where stent 241 is deployed in luminal aspect 140 comprising healthy tissue.

Additionally, the proximity of filter 122 to stent 241 ensures that a branch artery (not shown) cannot interpose between filter 122 and stent 241 to act as a conduit for debris 160 as would be the case if filter 122 was further away from stent 241. Other possible advantages of stent filter assembly 100 over existing technology accrue from stent 241, balloon 130 and filter 122 being deployed on single catheter 132, and include:
a) lower manufacturing-related costs for a single assembly 100 including stent 241 and filer 122 as compared to existing technology comprising separate filter and stent assemblies; and
b) lower surgical fees for performing a single procedure with single catheter 132 as compared to procedures associated with existing technology, noted above.

### Balloon, Stent and Filter Assembly 150

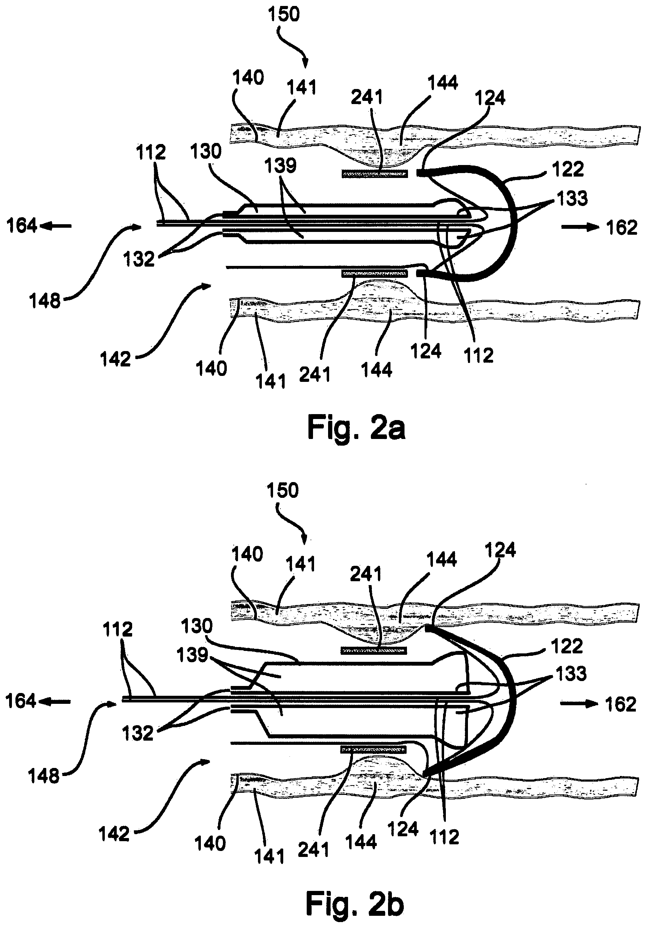

In one example, balloon 130 optionally comprises alternative shapes, for example having varied cross sectional diameters. As seen in an assembly 150 (Figure 2a), the diameter associated with a distal portion 133 of deflated balloon 130 is optionally larger than the diameter associated with a proximal portion 139.

In one example, filter 122 is separate from stent 241 and is pushed in direction 162 using cords 112 and/or distal balloon portion 133.

As seen in Figure 2b, filter 122 reaches a maximal diameter as distal balloon portion 133 fully inflates. In this manner, filter 122 is fully in position prior to inflation of proximal portion 139.

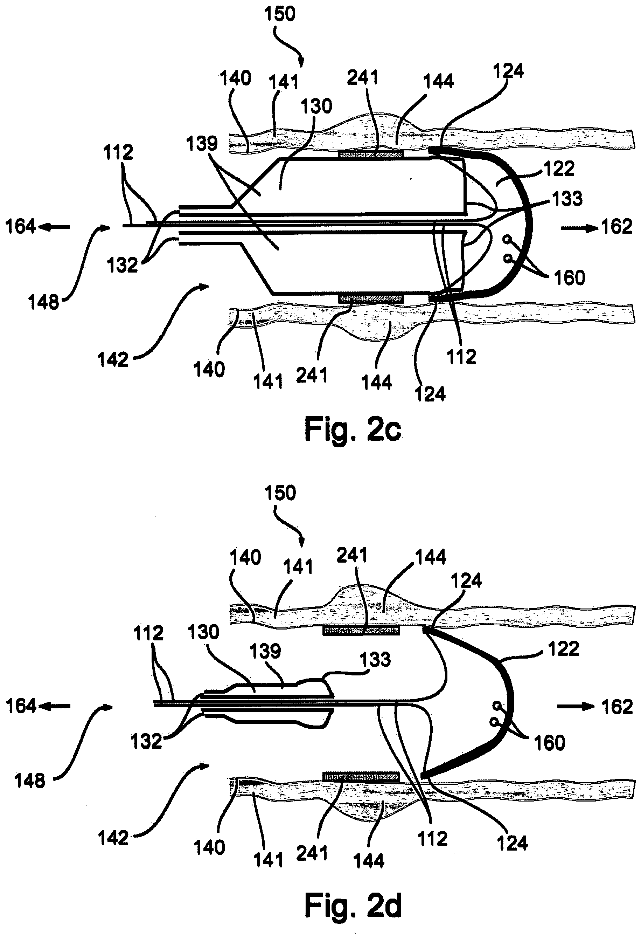

As seen in Figure 2c, further inflation of balloon 130 has caused proximal balloon portion 139 to fully inflate and radially expand stent 241. Radially expanded stent 241 compresses lesion 144 radially outwards to release debris 160 that is captured by filter 122.

In Figure 2d, balloon 130 has been deflated, leaving stent 241 and filter 122 in position against luminal aspect 140. In one example, filter 122 comprises materials and/or apertures that aid in removably connecting filter 122 to luminal aspect 140 so that filter 122 remains connected to luminal aspect 140 for a period of time after balloon 130 has deflated, herein contracted.

By remaining in contact with luminal aspect 140, filter 122 continues to filter debris 160 that may be released into lumen 142 from lesion 144 following radial expansion of stent 241. Filter 122 is contemplated to remain attached to luminal aspect 140 until danger of generation of debris 160 passes, for example between an hour and 24 hours. When filter 122 remains in position for an extended period, balloon 130 is optionally deflated and removed from lumen 142 while filter 122 and cords 112 are left in place.

In some examples, the type of the material and/or configuration of apertures in filter 122 ensure that filter 122 remains removably connected to luminal aspect 140 following deflation of balloon 130. In other examples, filter 122 includes a pressure sensitive adhesive having an affinity for luminal aspect 140 so filter 122 remains removably connected to vessel luminal aspect 140 following deflation of balloon 130.

There are many adhesives that may be contemplated for use in providing a removable connection of filter 122 to luminal aspect 140 including, *inter alia*: fibrin, biological glue, collagen, hydrogel, hydrocolloid, collagen alginate, and methylcellulose, to name a few.

Whether filter 122 comprises a mesh material alone or in combination with an adhesive, filter 122 is optionally configured to connect to luminal aspect 140 from pressure exerted by balloon 130 of, for example, between one and twenty atmospheres.

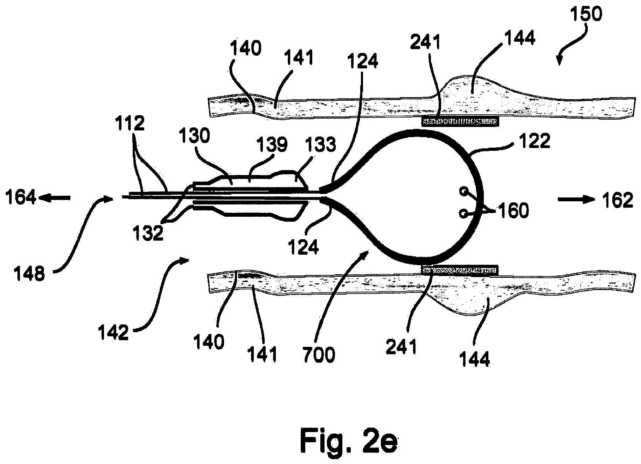

As seen in Figure 2e, filter opening 124 has been fully closed and filter 122 is passing through stent 241 on the way to the *ex vivo* environment.

### Self-Expanding Stent and Filter Assembly 400

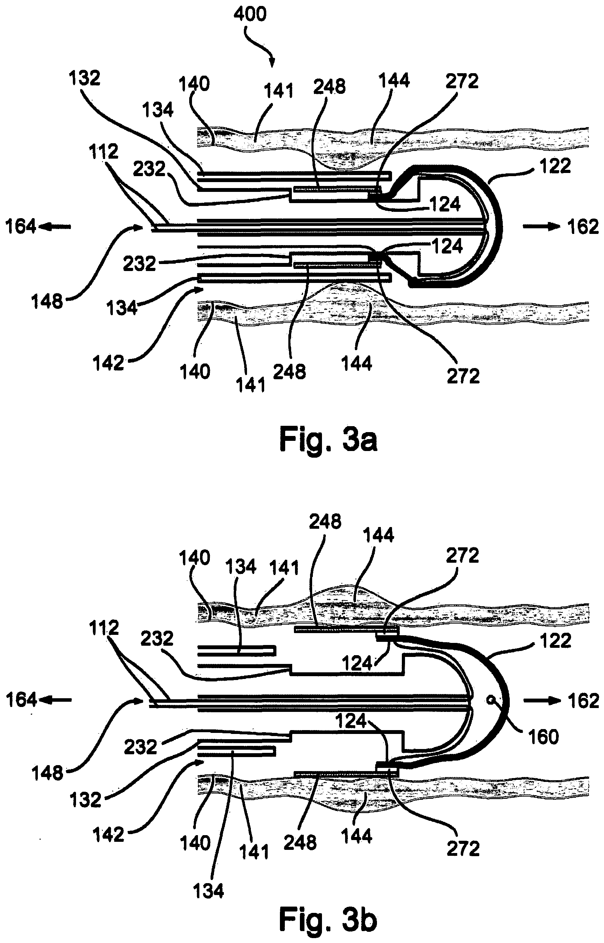

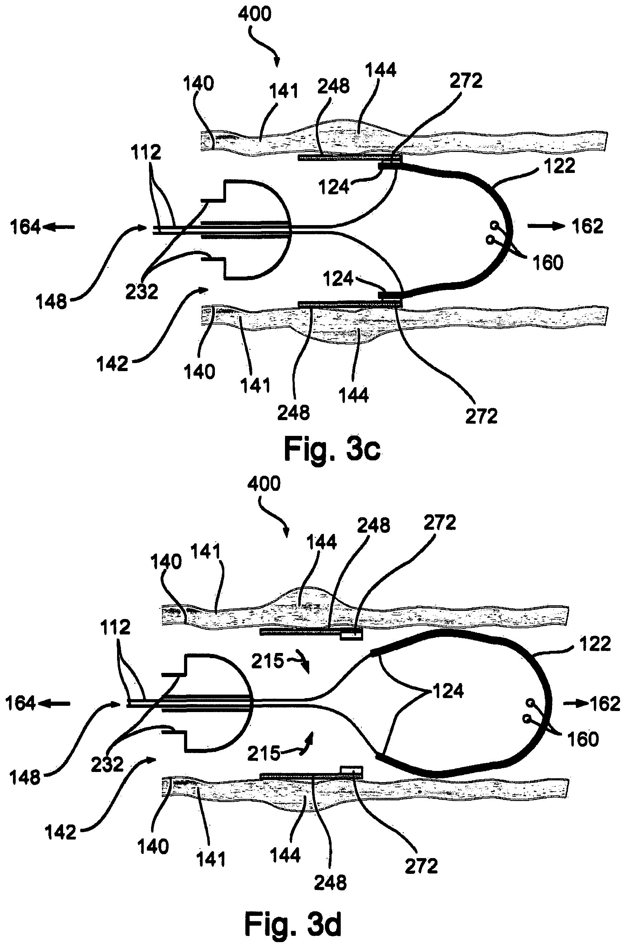

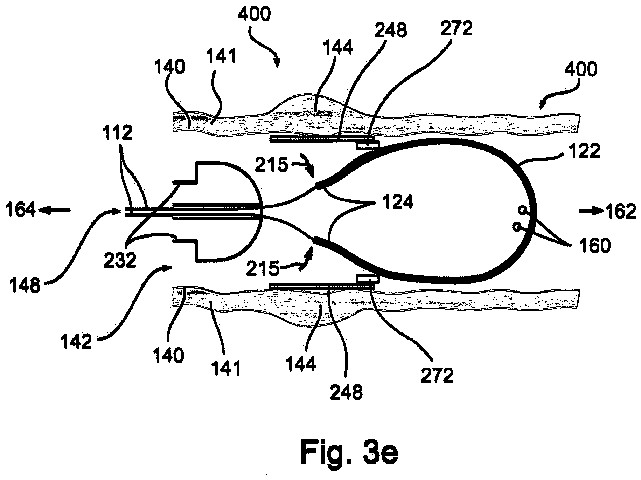

Assembly 400 (Figure 3 a) includes a self-expanding stent 248 in a contracted state on a spindle holder 232 extending from catheter 132. Stent 248 is initially maintained in a compressed, unexpanded, configuration against spindle 232 by a compression sleeve 134.

Self-expanding stent 248 includes adherent areas 272 that adhere distal portion of stent 248 to filter opening 124.

As seen in Figure 3b, compression sleeve 134 has been pulled proximally 164 to release stent 248 so that stent 248 expands radially outward, thereby crushing lesion 144.

As seen in Figure 3c, spindle 232 has been retracted from within filter 122 and self-expanding stent 248 and debris 160 has been captured in filter 122. In Figure 3d, cords 112 have been pulled proximally 164 through catheter channel 148, thereby causing filter opening 124 to disengage from adherent 272, contract radially inward 215 and pass through self-expanding stent 248 in direction 164.

While adherent areas 272 are shown as being attached to stent 248 and removably connected to filter 122, it is easily understood to those familiar with the art that adherent areas 272 optionally are attached to filter 122 and removably connected to stent 248.

While adherent areas 272 are shown as being internal to stent 248 and external to filter 122, it is easily understood to those familiar with the art that adherent areas 272 optionally are external to stent 248 and internal to filter 122.

As seen in Figure 3e, filter 122 is being drawn toward catheter channel 148. Thereafter, filter opening 124 is optionally closed as filter 122 passes through stent 248 on the way to the *ex vivo* environment.

In one example, stent 248 comprises an alloy that includes tantalum, tungsten, and zirconium: tantalum from about 20% to about 40% by weight; tungsten from about 0.5% to about 9% by weight; and zirconium from about 0.5% to about 10% by weight.

In an alternative example, self-expanding stent 248 comprises an alloy such as nitinol (Nickel-Titanium alloy), having shape memory characteristics.

Shape memory alloys have super-elastic characteristics that allow stent 248 to be deformed and restrained on spindle 232 during insertion through vessel 141. When compression sleeve 134 is removed (Figure 3b) and self-expanding stent 248 is exposed to the correct temperature conditions, the shape memory material returns to an original expanded configuration. Self-expanding stent 248, for example, is superelastic in the range from at least about twenty-one degrees Centigrade to no more than about thirty-seven degrees Centigrade.

As used herein, a nitinol alloy refers to an alloy comprising between about at least 50.5 atomic percent Nickel to no more than about 60 atomic percent Nickel with the remainder of the alloy being Titanium. The term nitinol is intended to refer to a two-component memory metal stent discussed above as well as any other type of known memory metal stent.

### Jacketed Stents 200, 300 and 390

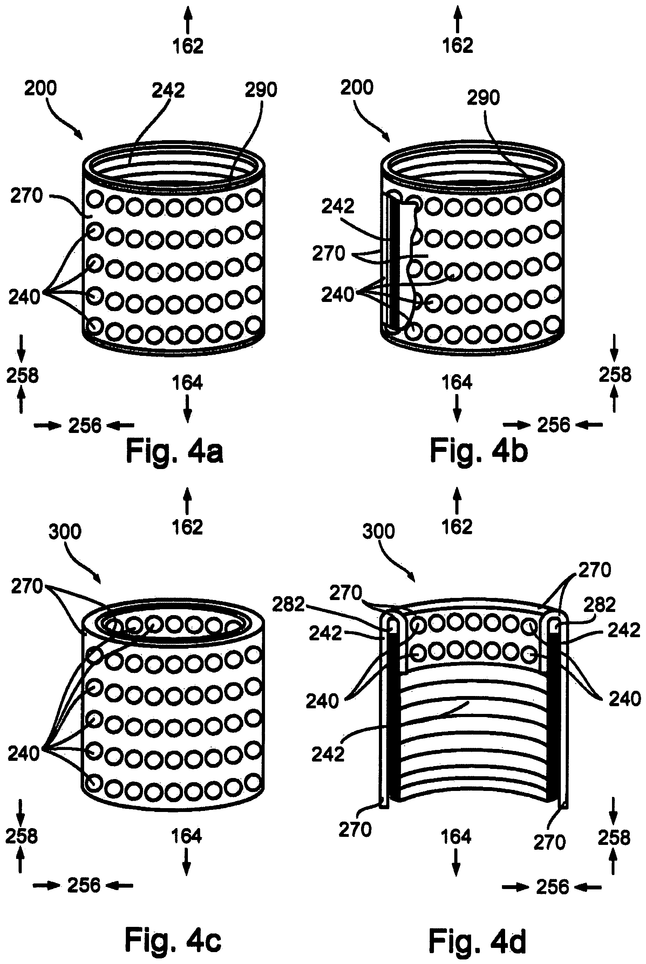

Figure 4a shows a jacketed stent 200 comprising an outer jacket 270 and an inner stent 242 that are connected by a distal connection 290. As seen in Figure 4b, besides distal connection 290, stent 242 and jacket 270 are substantially free of further connection.

During radially outward expansion in a direction 256, stent 242 typically contracts considerably in directions 258 while jacket 270 remains relatively stationary with respect to stent 242. Jacket 270 allows contraction of stent 242 while buffering shear forces generated by stent 242 on lesion 144 (Figure 1), thereby substantially preventing generation of unwanted and dangerous debris 160 during radial expansion.

In one example, distal connection 290 optionally comprises a process of sewing, adhesion, gluing, suturing, riveting and/or welding. Optionally, distal connection 290 is offset proximally 164 along stent 242, for example up to and including the center of stent 242 or along distal portion of stent 242.

Figures 4c and 4d show a jacketed stent 300 in which distal portion 162 of jacket 270 is folded over distal portion 162 of stent 242. Stent 242 is therefore substantially completely unattached to jacket 270. During radially outward expansion in direction 256, contraction of stent 242 in directions 258 results in gaps 282 between stent 242 and stentjacket 270 so that luminal vessel aspect 140 (Figure 1a) is buffered from shear forces generated by stent contraction in directions 258.

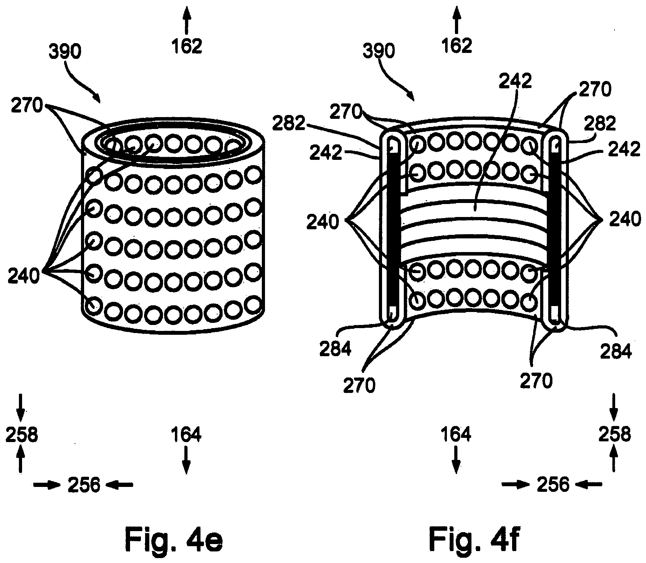

Figures 4e and 4f show still another example in which a jacketed stent 390 comprises jacket 270 that is folded over both the proximal 164 and distal 162 aspects of stent 242. Upon expansion of stent 242, distal gap 282 and/or a proximal gap 284 optionally form due to jacket 270 remaining substantially stationary with respect to contraction in directions 258 of stent 242.

In one example, jacket 270 is formed by a process including knitting, braiding, knotting, wrapping, interlacing, electrospinning and/or dipping a porous mold into one or more reagents.

In one example, jacket 270 is formed from one or more fibers having a diameter of between at least about 3 microns and no more that about 100 microns.

In one example, jacket 270 contains apertures 240 that substantially prevent generated stenotic debris and/or plaque associated with stenotic lesion 144 (Figure Ia) from entering apertures 240, thereby substantially preventing the above-noted tendency for plaque to be ripped from vessel luminal aspect 140. In one example, apertures have diameters of between at least about 20 microns and no more than about 200 microns. In one example, substantially all apertures 240 have substantially similar diameters. In another example, apertures 240 have variable diameters.

In one example, jacket 270 has a thickness of between at least about 20 microns and no more that about 200 microns.

In one example, unexpanded stent 242 has a diameter of at least about 0.3 millimeters and no more than about 3.0 millimeters; while expanded stent 242 has a diameter of at least about 1.0 millimeter to not more than about 8.0 millimeters.

In one example jacket 270 and/or stent 242 comprise materials that are coated and/or imbued with one or more active pharmaceutical agents for the purpose of preventing infection, inflammation, coagulation and/or thrombus formation.

Jacketed stents 200, 300 and 390 are optionally designed for use in a wide variety of vascular tissue including coronary, peripheral, cerebral, and/or carotid vascular tissue. Additionally, jacketed stents 200, 300 and 390 are optionally designed for use in treating an aortic aneurysm and/or a body lumen, for example a lumen associated with pulmonary tissue.

The many materials, manufacturing methods, uses and designs of jacket 270 and stent 242 are well known to those familiar with the art.

### Self-Expanding Jacketed Stent and Filter Assembly 500

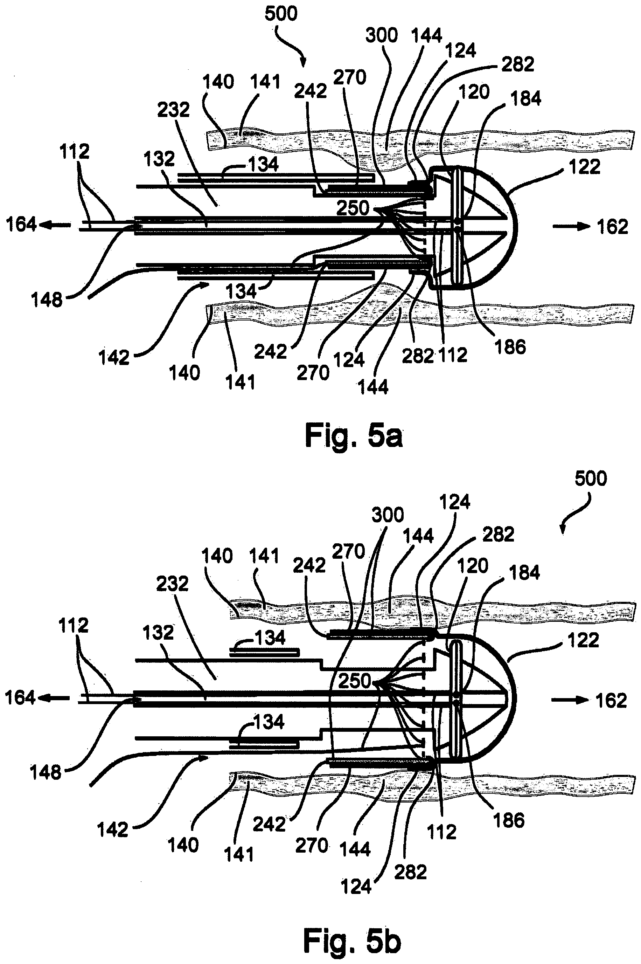

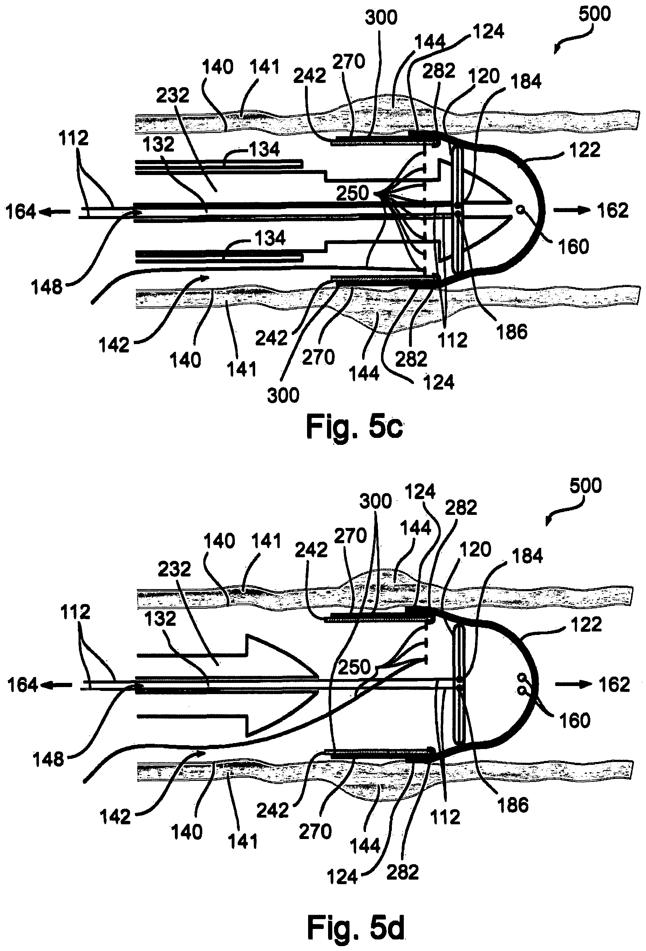

Figure 5a shows a jacketed stent and filter assembly 500 in accordance with an embodiment of the present invention comprising a removable belt 250 that is looped through filter 122 and jacket 270 and passes through compression sleeve 134 for manipulation by an *ex vivo* operator. In figures 5b and 5c, compression sleeve 134 is removed, allowing expansion of jacketed stent 300, as explained above. This will effect a radially outward compression of stenotic lesion 144.

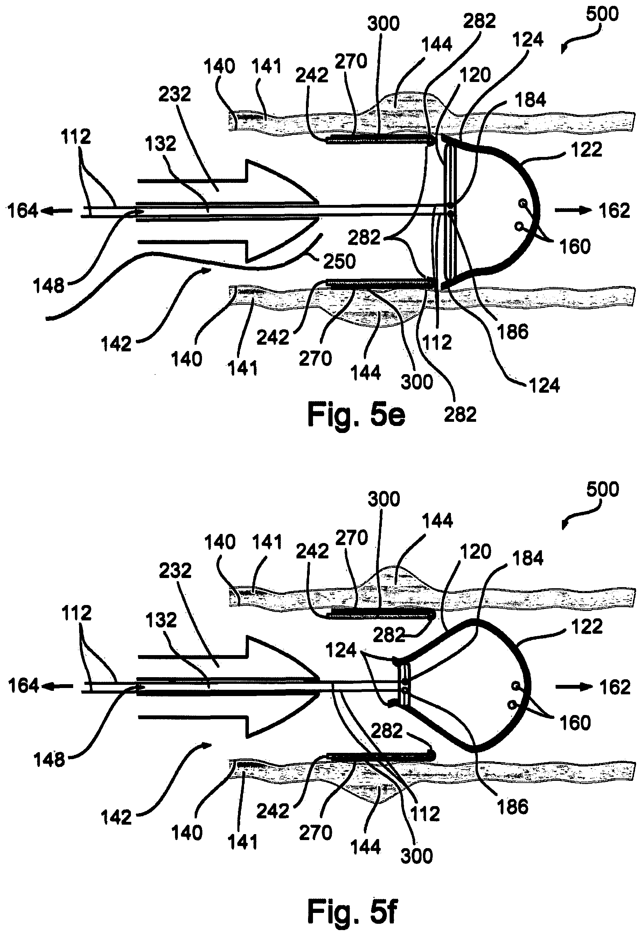

In Figure 5d, spindle holder 232 is retracted proximally 164, and removable belt 250 is pulled in direction 164 to free filter 122 from jacket 270. Figure 5e shows removable belt 250 fully disengaged from jacket 270 and filter 122, and debris 160 captured by filter 122.

While belt 250 is shown as being removably connected by running loops that pass through holes in jacket 270 and filter 122, there are many alternative removable connections contemplated. For example belt 250 optionally includes a series of hooks (not shown) that pass through apertures in jacket 270 and filter 122. Alternatively, belt 250 is optionally attached to a zipper-like mechanism that connects filter 122 to jacket 270; the many options for providing a removable connection between filter 122 and jacket 270 being easily understood by those familiar with the art.

In embodiments, cord 112 serves to cinch filter 122 closed. As shown, cord 112 passes distally 162 through channel 148 into a cinch channel 120, also referred to as guide channel 120 and cord channel 120, through a cord guide inlet 184. Cinch channel 120 guides cord 112 circumferentially around filter 122 until cord 112 exits cinch channel 120 through a cord outlet 186. Cord 112 then passes distally 162 through catheter channel 148 that passes through spindle 232 and catheter 132.

In this manner both ends of cord 112 exit catheter channel 148 and, by pulling both *ex vivo* ends of cord 112 proximally 164, filter 122 is contracted along cinch channel 120. As seen in Figure 2e, pulling of cords 112 proximally 164 has caused filter 122 to disconnect from luminal aspect 140.

As seen in Figure 5f, cords 112 have been pulled further proximally 164 to cause: collapse of filter 122, and capture of debris 160 generated by stenotic lesion 144. Filter 122 is then pulled into channel 148; and spindle 232 and filter 122 are removed from lumen 142.

While a single cord 112 and cinch channel 120 are shown, cinch channel 120 optionally comprises multiple pairs of inlets 184 and outlets 186, each associated with a separate cord 112. The many configurations and modifications of cinch channel 120, inlet 184, and outlet 186 are well known to those familiar with the art.

### Self-Expanding Jacketed Stent and Filter Assembly 600

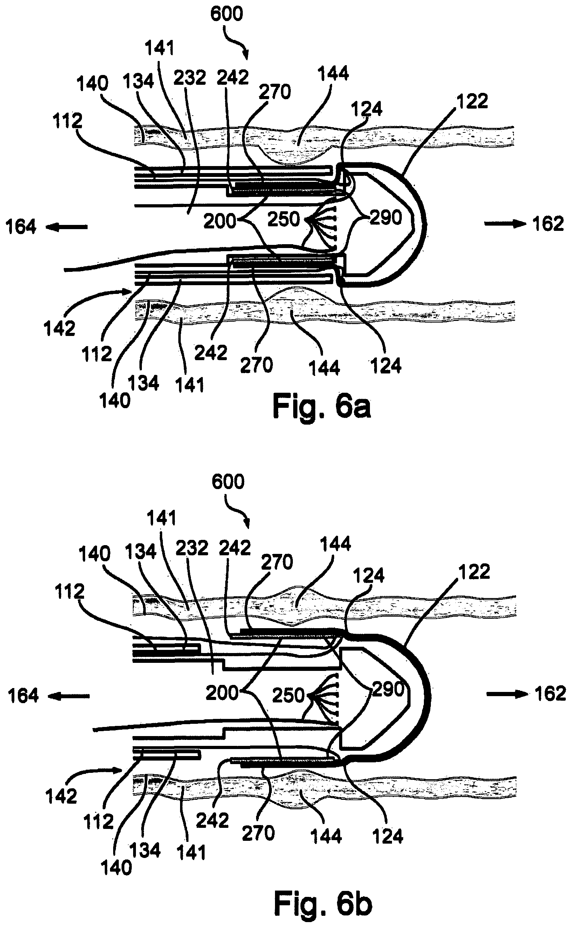

Figures 6a-6g show jacketed stent 600 in accordance with a further embodiment of the present invention in which cords 112 are connected to proximal portion 164 of filter 122 and pass internal to jacketed stent 600 and compression sleeve 134, along spindle 232. Removable belt 250 similarly passes internal to jacketed stent 600 and compression sleeve 134, and connects with filter 122 and jacket 270 just below connection 290.

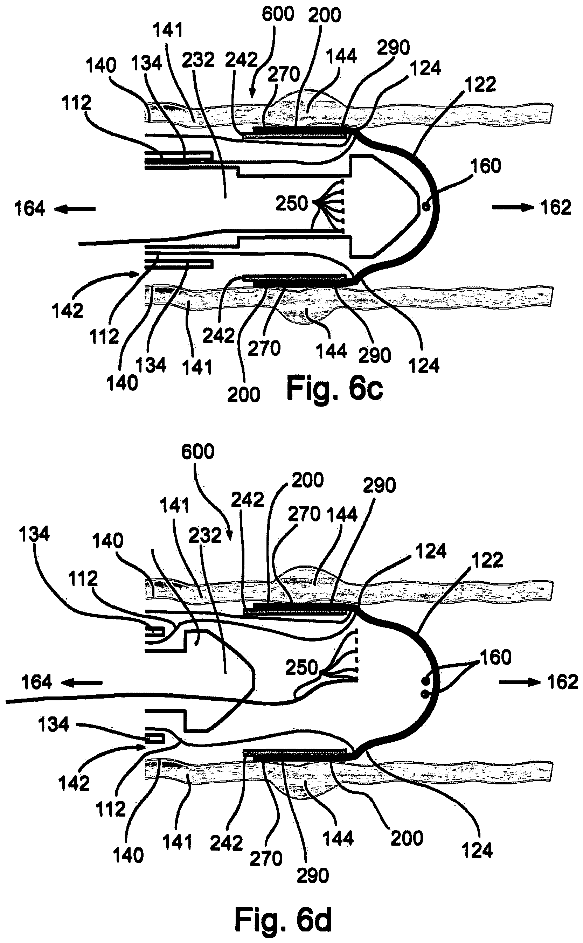

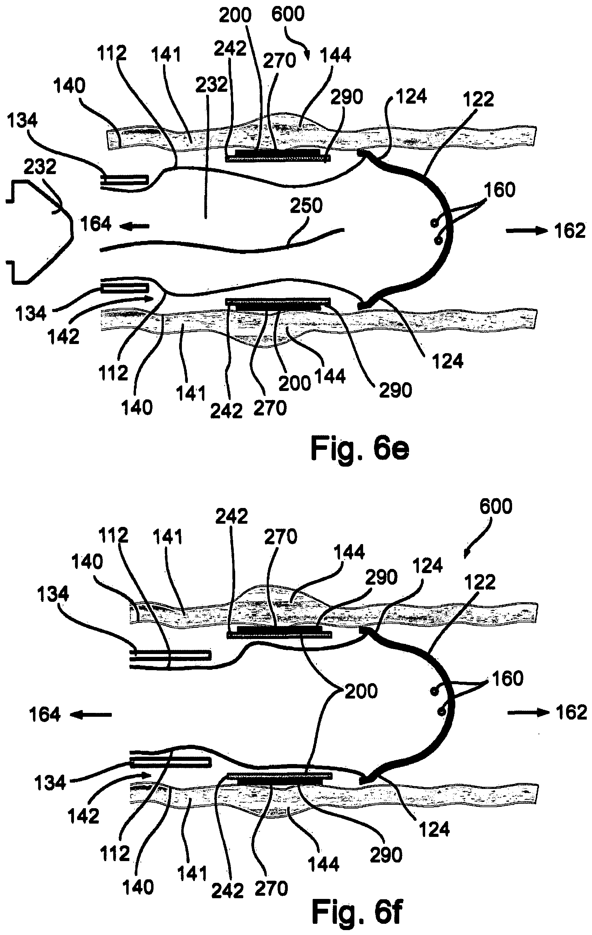

Following radial expansion of jacketed stent 600 (Figures 6b and 6c), as seen in Figure 6d; removable belt 250 is pulled proximally 164. As seen in Figures 6e and 6f, following complete removal of belt 250, filter 122 is disengaged from jacketed stent 200 and spindle 232 is removed from lumen 142.

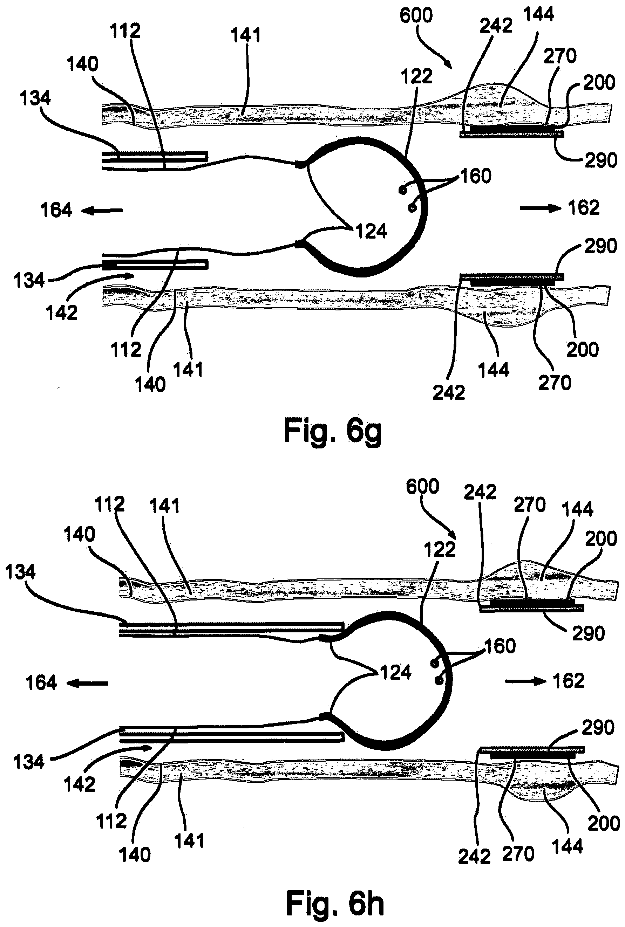

In Figure 6g, filter 122 is pulled proximally 164 by cords 112 through stent 242. Cords 112 are pulled in direction 164 while compression sleeve 134 remains substantially stationary, causing filter 122 to enter compression sleeve 134, as seen in Figure 6h. Alternatively, compression sleeve 134 is advanced in direction 162 while cords 112 are held substantially stationary.

Filter 122 is shown partially pulled into compression sleeve 134 and compression sleeve 134 and filter 122 are now pulled in tandem, proximally 164 for percutaneous removal from lumen 142.

In embodiments, filter 122 is pulled completely into compression sleeve 134 so that compression sleeve 134 serves as a housing for filter 122 to prevent filter 122 from rubbing against luminal aspect 140 during removal from lumen 142.

### Filter Assembly 1100

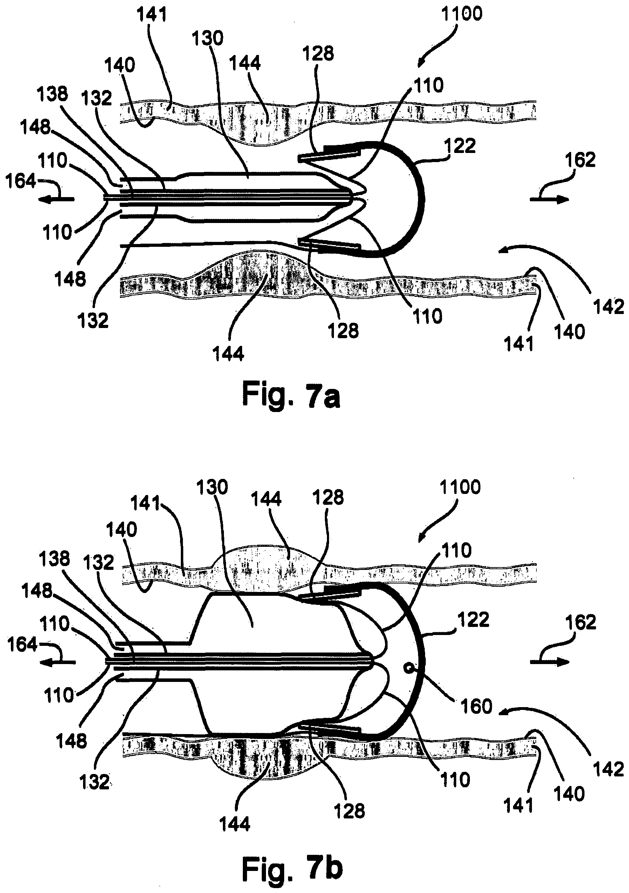

Figure 7a shows an exemplary representation of an *in vivo* debris filter assembly 1100 in a cross section of a blood vessel 141. Filter 122 is shown in a contracted, pre-dilated, position with loose cords 110 attached to two struts 128 that are connected to filter 122. Cords 110 exit filter 122 and pass through a lumen 138 and into and through catheter 132. Cords 110 typically exit lumen 138 *ex vivo,* thereby allowing *ex vivo* manipulation by an operator.

A balloon 130 projects downstream of catheter 132 and is positioned adjacent to stenotic lesion 144. Balloon 130 typically comprises a biologically compatible elastomeric material, or semi compliant material, for example: rubber, silicon rubber, latex rubber, polyethylene, polyethylene terephthalate, Mylar, and/or polyvinyl chloride.

In Figure 7b, balloon 130 has been inflated by introducing fluid through a fluid channel 148 that is substantially coaxial to catheter 132. During inflation of balloon 130, after the diameter of balloon 130 reaches the distance between struts 128, continued inflation of balloon 130 causes struts 128 to bias radially outwardly, thereby expanding filter 122.

Once inflated, filter 122 filters debris 160 that is released from stenotic lesion 144 and continues to filter debris 160 even as balloon 130 is deflated, as explained below.

While filter 122 is shown in an expanded position as a generally curved structure, balloon 130 may alternatively have a variety of shapes, including a conus having an apex located downstream of balloon 130.

Filter 122 typically comprises a mesh sheet material that is configured to filter debris 160 from lumen 142. Filter 122 typically includes apertures having diameters of between at least about 20 microns and no more than about 200 microns in diameter.

Additionally, filter 122 and/or struts 128, are configured to flex outward until such flexion is limited by a luminal aspect 140, for example a diameter of between 3.0 and 6.0 millimeters, depending on the size of lumen 142 in which filter 122 is deployed.

In one example, portions of filter 122 and/or struts 128 comprise superelastic material, for example nitinol; an elastic material; and/or a plastic material; the many materials and their properties being well-known to those familiar with the art.

Similarly, balloon 130 has an inflation diameter of between 3.0 and 6.0 millimeters, depending on the cross sectional diameter of lumen 142. In larger vessels 141, balloon 130 and filter 122 optionally are manufactured to have larger maximal diameters. In smaller vessels, for example to cut down on the bulk of deflated balloon 130 and filter 122, smaller maximal diameters are optionally appropriate.

Filter 122 comprises materials and/or apertures that aid in removably connecting filter 122 to an *in vivo* luminal aspect 140. In this manner, filter 122 remains connected to luminal aspect 140 for a period of time after balloon 130 has deflated, herein contracted, by egress of fluid through channel 148. By remaining in contact with luminal aspect 140, filter 122 continues to filter debris 160 that may be released into lumen 142 from lesion 144 while balloon 130 is in a contracted state.

In some examples, the material and configuration of filter 122 ensures that filter 122 remains removably connected to luminal aspect 140 following deflation of balloon 130. In other examples, filter 122 includes a pressure sensitive adhesive having an affinity for luminal aspect 140 so that the adhesive, optionally in conjunction with the material of filter 130, remain removably connected to vessel luminal aspect 140 following deflation of balloon 130.

There are many adhesives that may be contemplated for use in providing a removable connection of filter 122 to luminal aspect 140 including, *inter alia*: fibrin, biological glue, collagen, hydrogel, hydrocolloid, collagen alginate, and methylcellulose, to name a few.

Whether filter 122 comprises a mesh material alone or in combination with an adhesive, filter 122 is optionally configured to removably connect to luminal aspect 140 from pressure exerted by balloon 130 of, for example, between one and twenty atmospheres.

In further arrangements, for example when there is continued danger of debris 160 being generated after lesion 144 has been compressed, balloon 130 is optionally deflated and removed from lumen 142, while filter 122 is left in place. Filter 122 optionally is left connected to luminal aspect 140 by the configuration of filter 122 and/or biological glues noted above until the danger of generation of debris 160 has passed.

As noted above, during a typical balloon angioplasty, balloon 130 is sequentially inflated to a pressure of several atmospheres and deflated. In one example, filter 122 remains removably connected to luminal aspect 140 following the first inflation of balloon 130 and throughout several sequences of inflation and deflation.

As filter 122 is deployed relatively proximate to lesion 144 where luminal aspect 140 generally comprises unhealthy tissue, the chance that filter 122 will cause damage to healthy tissue of luminal aspect 140 is very low.

Additionally, the proximity of filter 122 to balloon 130 substantially lowers the odds that a branch artery will be located between filter 122 and balloon 130, to act as a conduit for debris 160. Further, as balloon 130 and filter 122 are deployed on single catheter 132, the cost for each assembly 1100 should be lower than existing technology employing a separate filter. Moreover, as assembly 1100 includes balloon 130 and filter 122 mounted on a single catheter, the complexity of manufacture, deployment and the surgical fees to the surgeon should be reduced over existing technology.

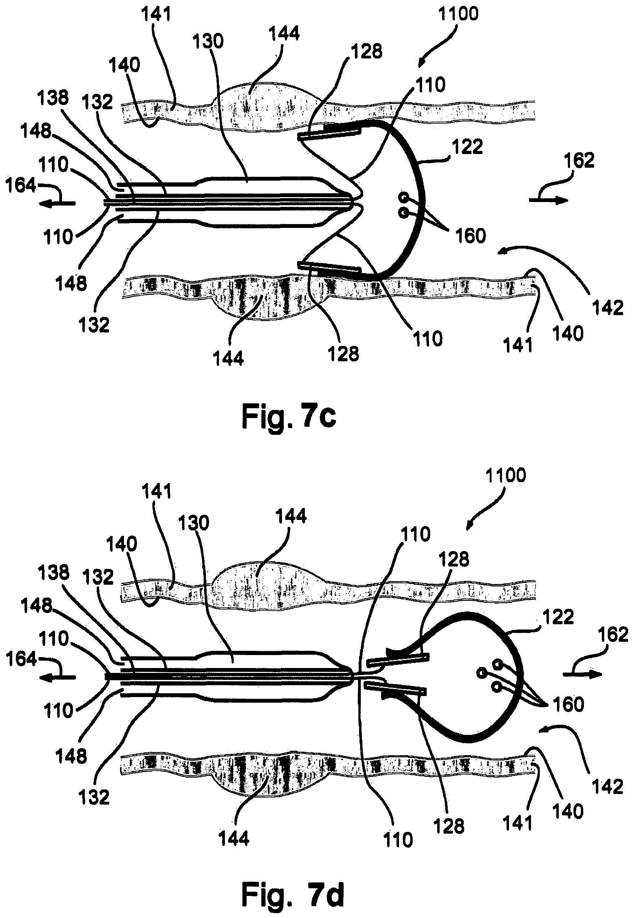

As seen in Figure 7c, after stenotic lesion 144 has been cracked and squashed radially outwards, balloon 130 is deflated and filter 122 remains in an expanded state and continues to capture debris 160. As the fluid contained in lumen 142 is moving in a direction 162, in a distal or downstream direction with respect to filter 122, debris 160 remains in place, captured within filter 122.

To disconnect filter 122 from luminal aspect 140, cords 110 are pulled proximally, upstream, in direction 164. Cords 110 are then pulled further to cause filter 122 to enclose debris 160 as seen in Figure 7d.

While cords 110, as shown, pass through catheter lumen 138, in alternative arrangements, cords 110 pass to the side of balloon 130 without passing through lumen 138. Further, while balloon 130 is shown attached to catheter 132, there are many alternative options for delivering balloon 130 and filter 122, for example using a guide wire. Those familiar with the art will readily recognize the many alternative modes and configurations available for delivery and operation of balloon 130 and filter 122.

In one example, filter 122 is configured to disconnect from luminal aspect 140 in response to tension applied to cords 110 of at least about one Newton and no more than about 20 Newtons.

As the diameter of lumen 142 is larger than the diameter of catheter lumen 138, continued upstream pull in direction 164 on cords 110, biases the proximal portions of struts 128 radially inward, causing the proximal edges of filter 122 to move radially inward so that filter 122 disconnects from luminal aspect 140. Following disconnection of filter 122 from luminal aspect 140, continued pulling of cords 110 in direction 164 causes struts 128 to inwardly bias, thereby reducing the upstream cross sectional diameter of filter 122.

As the fluid in lumen 142 travels distally in direction 162, pulling catheter 132 and filter 122 in proximal direction 164 causing debris 160 to move downstream against filter 122 so that debris 160 remains captured by filter 122.

Thus, filter 122 maintains captured debris 160 even when there is a distance between struts 128, as might occur when there is considerable volume of debris 160, for example in large arteries. Optionally, cords 110 are pulled in direction 164 until a portion of filter 122 contacts balloon 130 and/or enters catheter lumen 138.

While two struts 128 are shown connected to two cords 110, the present examples contemplate four or even eight struts 128, with each strut 128, or each pair of struts 128, being attached to individual cords 110 that remove filter 122 from luminal aspect 140.

Alternatively, assembly 1100 contemplates using a single strut 128 with a single cord 110 connected to it that encircles filter 122 and slidingly attaches to strut 128 in a lasso configuration. Pulling on single cord 110 causes contraction of struts 128 and of the associated cross-sectional circumference of filter 122, thereby preventing egress of debris 160 filter 122. The many options available for configuring cords 110 and struts 128 to effectively close filter 122 are well known to those familiar with the art.

### Filter Assembly 1200

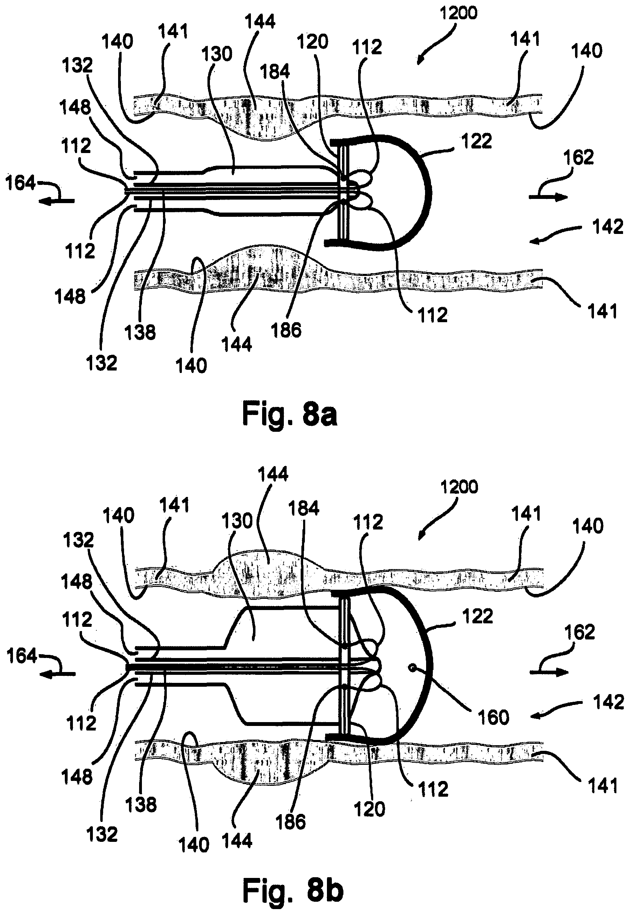

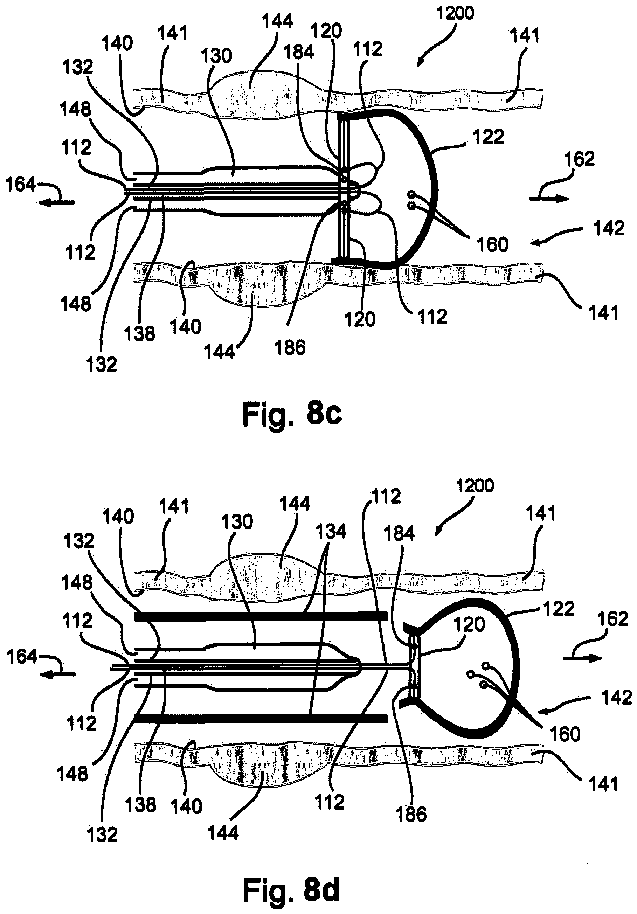

Figure 8a shows an example of an assembly 1200 in which single cord 112 passes distally in direction 162 through catheter lumen 138. Cord 112 then curves within filter 122 to pass in a proximal direction 164 into a cord inlet 184 and through cord channel 120. Cord channel 120 guides cord 112 circumferentially around filter 122. After circling filter 122, cord 112 exits channel 120 through cord outlet 186 and passes distally in direction 162 into filter 122. Cord 112 then curves within filter 122 to pass in a proximal direction 164 into and through catheter lumen 138.

In this manner both ends of cord 112 exit catheter lumen 138 and, by pulling both *ex vivo* ends of cord 112 in direction 164, filter 122 is contracted along cord channel 120, as seen in Figure 8d.

While a single cord 112 is shown, channel 120 optionally comprises multiple pairs of inlets 184 and outlets 186, each associated with a separate cord 112. The many configurations and modifications of channel 120, inlet 184, and outlet 186 are well known to those familiar with the art.

Figure 8d shows an example of a tubular compression sleeve 134 that is coaxial with catheter 132. Sleeve 134 has been slidingly pushed through vessel lumen 142 in direction 162 until sleeve 134 approaches filter 122.

In one example, pulling cord 112 and/or catheter 132 in direction 164 while holding sleeve 134 substantially stationary, pulls filter 122 into compression sleeve 134. Alternatively, compression sleeve 134 is advanced in direction 162 while catheter 132 and/or cord 112 are held substantially stationary.

In one example, compression sleeve 134 serves as a housing for filter 122 to prevent filter 122 from scraping along luminal aspect 140 during removal from lumen 142. Additionally or alternatively, compression sleeve 134 serves to compress filter 122 into a smaller maximal circumferential diameter so that filter 122 more easily passes through lumen 142 during removal of filter 122.

### Balloon Assembly 1300

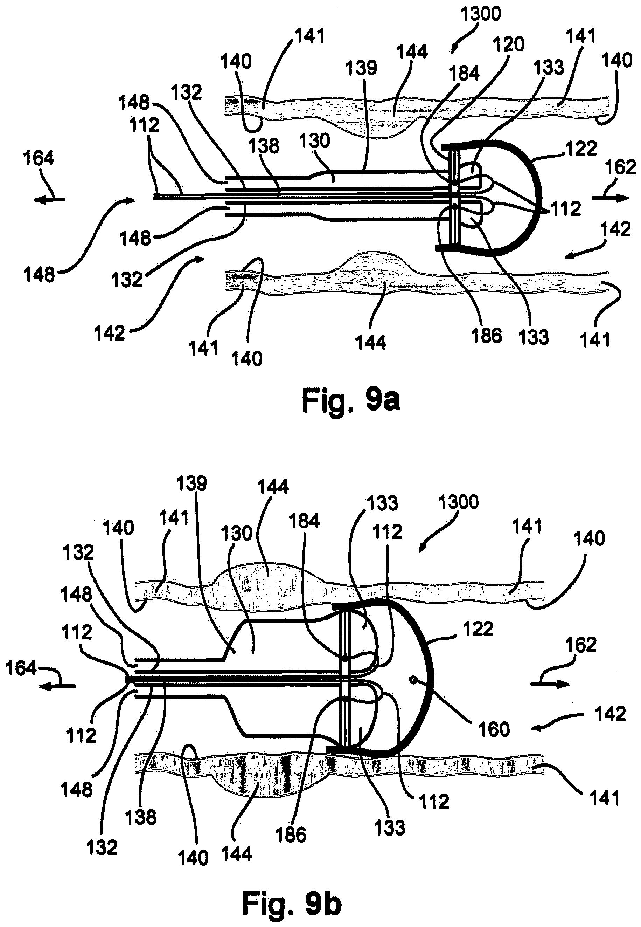

In one example, balloon 130 optionally includes alternative shapes, for example having varied cross sectional diameters. As seen in assembly 1300 (Figure 9a), the diameter associated with distal portion 133 of deflated balloon 130 is larger than the diameter associated with proximal portion 139.

As seen in Figure 9b, filter 122 reaches a maximal diameter initially as distal balloon portion 133 inflates. In this manner, filter 122 is fully in position and expanded prior to inflation of proximal balloon portion 139.

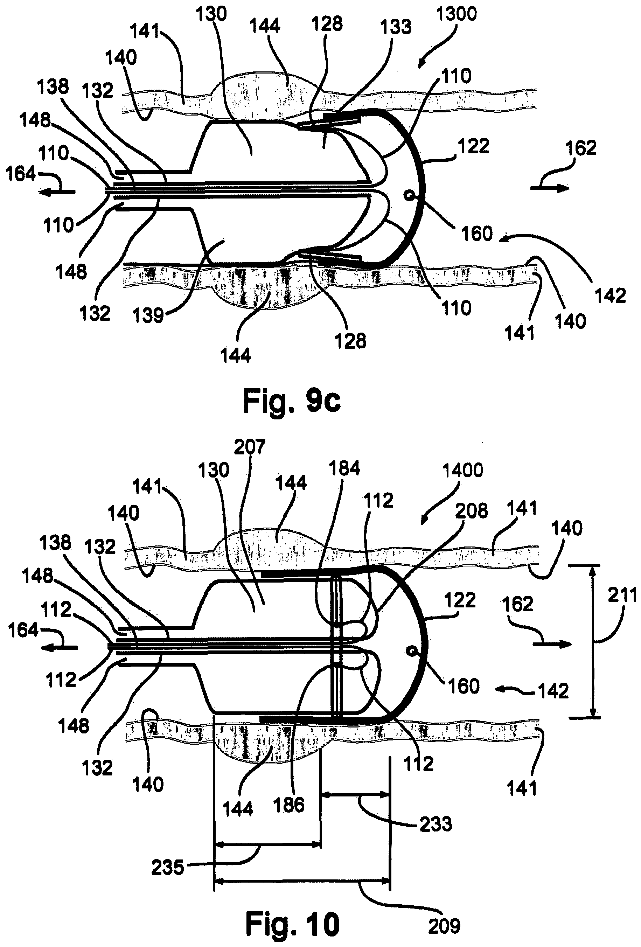

As seen in Figure 9c, proximal balloon portion 139 has been fully inflated to compress lesion 144, thereby releasing debris 160 that is captured by filter 122. The many options for configuring alternative shapes of balloon 130 are well known to those familiar with the art.

### Balloon and Filter Assembly 1400

There are additionally many methods of assembling filter 122 and balloon 130, as seen in assembly 1400 (Figure 10). In one example, balloon 130 is seen having an overall length 209 of approximately 38 millimeters and a maximal inflation diameter 211 of approximately 5 millimeters.

Additionally, balloon 130 is shown with a proximal portion 207 having a length 235 of approximately 18 millimeters and a distal portion 208 having a length 233 of approximately 18 millimeters.

In one example, filter 122 extends to substantially cover distal portion 208 while proximal portion 207 is unprotected by filter 122.

In alternative configurations of assembly 1400, filter 122 optionally substantially fully covers distal balloon portion 208 and extends over at least a portion of proximal balloon portion 207; the many configurations of assembly 1400 being well known to those familiar with the art.

### Dual Balloon Assembly 1500

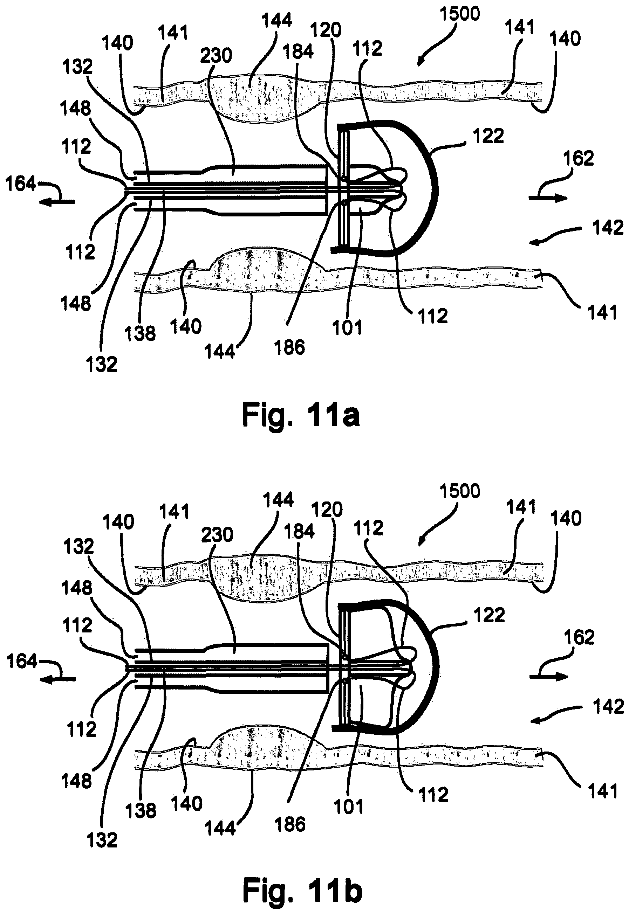

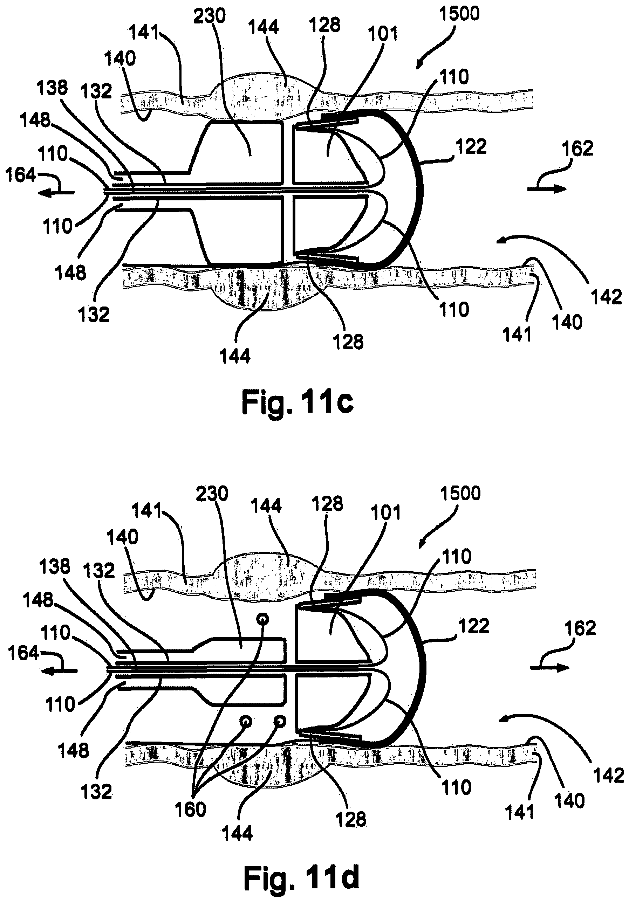

Assembly 1500 (Figures 11a-11e) comprises a proximal balloon 230 and a distal balloon 101. As seen in Figure 11b, distal balloon 101 is inflated to expand filter 122 and substantially take up the volume within filter 122. As seen in Figure 11c, proximal balloon 230 is inflated separately and pressed against lesion 144.

After deflation of proximal balloon 230 as seen in Figure 11d, distal balloon 101 remains inflated so that debris 160 remains proximal to distal balloon 101. Upon deflation of distal balloon 101, debris 160 enters and is captured by filter 122.

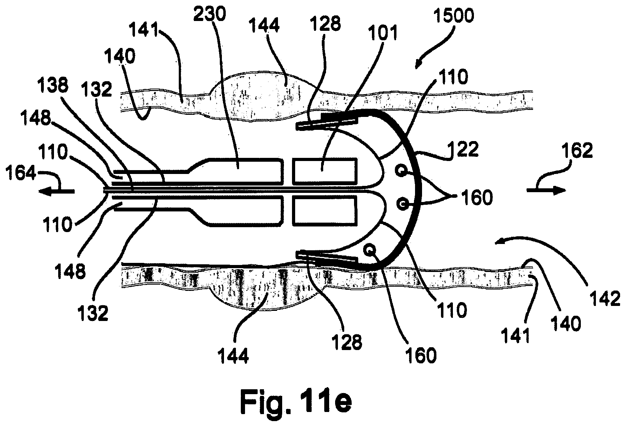

As seen in Figure 11e, distal balloon 101 is then deflated so that debris 160 is captured as filter 122 closes.

### Alternative Environments

While assemblies 1100-1500 have been described with respect to vessel 141, assemblies 1100-1500 can be easily configured for use in a wide variety of *in vivo* lumens 142 including *inter alia*: a lumen of a urethra, a biliary lumen and/or a renal calyx lumen. Additionally or alternatively, filter 122 can be easily modified to capture debris in virtually any *in vivo* lumen 142 including, *inter alia*: biliary stones and/or renal stones. The many applications, modifications and configurations of assemblies 1100-1500 for use in virtually any *in vivo* lumen 142 will be readily apparent to those familiar with the art.

### Materials and Design

In one example, the sheet material of filter 122 is selected from the group consisting of meshes and nets.

In one example, bending of a portion of the sheet material of filter 122 forms cinch channel 120. In one example, attaching a shaped component to filter 122 forms cinch channel 120 (Figure 5a).

In one example, filter 122 is configured to expand to a cross sectional diameter of at least about 1.0 millimeters. In one example, filter 122 is configured to expand to a cross sectional diameter of no more than about 6.0 millimeters. In one example, the extent of the expansion of filter 122 is configured to be limited by the walls of luminal aspect 140 in which filter 122 is deployed.

In one example, balloon 130 (Figure 1a) has a maximum inflation diameter of at least about 1.0 millimeter. In one example, balloon 130 has a maximum inflation diameter of no more than about 6.0 millimeters.

In one example, balloon 130 has a wall thickness of at least about 0.2 millimeters. In one example, balloon 130 has a wall thickness of no more than about 0.5 millimeters.

In one example, filter 122 (Figure 6a) has an internal surface that is attached to an external aspect of stent 242 and/or jacket 270. Alternatively, filter 122 has an external surface that is attached to an internal aspect or distal portion 162 of stent 242 and/or jacket 270.

In one example, catheter 132 (Figure 1a) has an outside diameter of at least about 1.0 millimeter. In one example, catheter 132 has an outside diameter of no more than about 5.0 millimeters. In one example, catheter 132 has a length of at least about 0.8 meter. In one example, catheter 132 has a length of no more than about 1.5 meters.

In one example, the walls of catheter 132 and compression sleeve 134 (Figure 3a) have a thickness of at least about 2 millimeters. In one example, the walls of catheter 132 and compression sleeve 134 have a thickness of more than about 5 millimeters.

In embodiments, jacket 270, (Figure 5a) filter 122, cord 112, compression sleeve 134, spindle 232 and catheter 132, comprise materials from the group consisting of: polyethylene, polyvinyl chloride, polyurethane and nylon.

In embodiments, jacket 270, filter 122, cord 112, compression sleeve 134, spindle 232 and catheter 132, comprise a material selected from the group consisting of: nitinol, stainless steel shape memory materials, metals, synthetic biostable polymer, a natural polymer, and an inorganic material. In embodiments, the biostable polymer comprises a material from the group consisting of: a polyolefin, a polyurethane, a fluorinated polyolefin, a chlorinated polyolefin, a polyamide, an acrylate polymer, an acrylamide polymer, a vinyl polymer, a polyacetal, a polycarbonate, a polyether, a polyester, an aromatic polyester, a polysulfone, and a silicone rubber.

In embodiments the natural polymer comprises a material from the group consisting of: a polyolefin, a polyurethane, a Mylar, a silicone, and a fluorinated polyolefin.

In embodiments, jacket 270, filter 122, cord 112, compression sleeve 134, spindle 232 and catheter 132, comprise materials having a property selected from the group consisting of: compliant, flexible, plastic, and rigid.

In one example, balloon 130 comprises a biologically compatible elastomeric material, or semi-compliant material, for example: rubber, silicon rubber, latex rubber, polyethylene, polyethylene terephthalate, Mylar, and/or polyvinyl chloride.

Balloon 130 typically has an inflation diameter of between 3.0 and 6.0 millimeters, depending on the cross sectional diameter of lumen 142. In larger vessels 141, balloon 130 and filter 122 optionally are manufactured to have larger maximal diameters. In smaller vessels, for example to reduce the bulk of contracted stent 241 and filter 122, smaller maximal diameters, hence less reduced material in stent 241 and filter 122, may be contemplated.

While balloon 130 is shown attached to catheter, 132, there are many alternative options for delivering balloon 130 and filter 122, for example using a guidewire. Those familiar with the art will readily recognize the many alternative modes and configurations available for delivery and operation of balloon 130 and filter 122.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

Accordingly, the invention is intended to embrace all such alternatives, modifications and variations that fall within the scope of the appended claims.

## Claims

1. A filter assembly (500)(600) for collecting debris from a stenotic vessel, comprising:
a) a radially expandable vascular flow-increasing device comprising a radially expandable stent (300)(200) configured to open a stenotic lumen, said radially expandable stent (300)(200) having a proximal end, a distal end and a lumen connecting said proximal and said distal ends; and
b) a filter (122) including an expandable proximal opening (124) having an operative connection with said radially expandable vascular flow-increasing device, said proximal opening (124) configured to expand in conjunction with expansion of said device, such that when said opening (124) is in an expanded configuration, said filter (122) is configured to filter debris from a fluid stream in which said filter is disposed;
wherein the filter (122) is configured to be disengaged from said expandable vascular flow-increasing device and removed from the stenotic vessel while the radially expandable stent (300)(200) remains in the stenotic vessel;
**characterised in that** said radially expandable vascular flow-increasing device further comprises:
a jacket (270) substantially surrounding an exterior surface of said expandable stent (300)(200), said jacket (270) configured to expand in conjunction with expansion of said expanding stent (300)(200); and
said operative connection comprises an operative connection between said filter (122) and said jacket (270).

2. The assembly (500)(600) according to claim 1, wherein said radially expandable vascular flow-increasing device comprises:
a) at least one balloon (130) configured to volumetrically expand and, during at least a portion of said expansion, operatively connect with said filter (122), and to contract following said expansion; and
b) said operative connection comprises an operative connection between said filter (122) and said at least one balloon (130) during at least a portion of said volumetric expansion of said at least one balloon (130).

3. The assembly (500)(600) according to claim 2, wherein at least a portion of said filter (122) is configured to remain removably connected to a luminal aspect during said contraction of said at least one balloon (130).

4. The assembly (500)(600) according to claim 2, wherein at least a portion of said filter (122) includes a pressure-sensitive adhesive having an affinity for a tissue associated with an in vivo luminal aspect.

5. The assembly (500)(600) according to claim 4, including a compression sleeve (134) comprising a substantially curved wall having a proximal end, a distal end and a lumen extending from said proximal end to said distal end, said lumen having a cross sectional diameter that is substantially smaller than the maximal cross sectional diameter of said luminal aspect.

6. The assembly (500)(600) according to claim 1, wherein said filter (122) includes a distal portion, a proximal portion, an opening to said filter (122) associated with said proximal portion and at least one strut operatively associated with said proximal portion.

7. The assembly (500)(600) according to claim 1, wherein said filter (122) includes: a) a distal portion; b) a proximal portion; c) an opening to said filter associated with said proximal portion; and d) at least one cord guide channel (120) circumferentially encircling at least a portion said proximal portion.

8. The assembly (500)(600) according to claim 1, wherein said filter (122) comprises a material having tissue connective properties for a portion of luminal tissue associated with an in vivo fluid stream.

9. The assembly (500)(600) according to claim 1, including a stent holding spindle (232) operatively associated with said stent (300)(200) when said stent (300)(200) is in a contacted configuration, said spindle (232) being mounted on a distal portion of an elongate catheter.

10. The assembly (500)(600) according to any of the preceding claims, wherein the stent (300)(200) includes a metallic base and the filter (122) is formed of a synthetic biostable polymer or a natural polymer.

11. The assembly (500)(600) according to any of the preceding claims, wherein the stent (300)(200) is configured to substantially support the proximal opening (124) of the filter (122) during expansion of the stent (300)(200).

12. The assembly (500)(600) according to any of the preceding claims, wherein the filter (122) is configured to flex outward until limited by a luminal aspect.

## Patentansprüche

1. Filterbaugruppe (500)(600) zum Sammeln von Debris von einem stenotischen Gefäß, die Folgendes umfasst:
a) eine radial ausdehnbare Gefäßflussverstärkungsvorrichtung, die einen radial ausdehnbaren Stent (300)(200) umfasst, konfiguriert zum Öffnen eines stenotischen Lumens, wobei der genannte radial ausdehnbare Stent (300)(200) ein proximales Ende, ein distales Ende und ein Lumen hat, das das genannte proximale und das genannte distale Ende miteinander verbindet; und
b) ein Filter (122) mit einer ausdehnbaren proximalen Öffnung (124) mit einer operativen Verbindung mit der genannten radial ausdehnbaren Gefäßflussverstärkungsvorrichtung, wobei die genannte proximale Öffnung (124) zum Ausdehnen in Verbindung mit der Ausdehnung der genannten Vorrichtung konfiguriert ist, so dass, wenn die genannte Öffnung (124) in einer ausgedehnten Konfiguration ist, das genannte Filter (122) zum Filtern von Debris aus einem Fluidstrom konfiguriert ist, in dem das genannte Filter angeordnet ist;
wobei das Filter (122) zum Lösen von der genannten ausdehnbaren Gefäßflussverstärkungsvorrichtung und zum Entfernen von dem stenotischen Gefäß konfiguriert ist, während der radial ausdehnbare Stent (300)(200) in dem stenotischen Gefäß verbleibt;
**dadurch gekennzeichnet, dass** die genannte radial ausdehnbare Gefäßflussverstärkungsvorrichtung ferner Folgendes umfasst:
einen Mantel (270), der eine Außenfläche des genannten ausdehnbaren Stents (300)(200) im Wesentlichen umgibt, wobei der genannte Mantel (270) zum Ausdehnen in Verbindung mit der Ausdehnung des genannten ausdehnbaren Stents (300)(200) konfiguriert ist; und
die genannte operative Verbindung eine operative Verbindung zwischen dem genannten Filter (122) und dem genannten Mantel (270) umfasst.

2. Baugruppe (500) (600) nach Anspruch 1, wobei die genannte radial ausdehnbare Gefäßflussverstärkungsvorrichtung Folgendes umfasst:
a) wenigstens einen Ballon (130), konfiguriert zum volumetrischen Ausdehnen und, während wenigstens eines Teils der genannten Ausdehnung, zum operativen Verbinden mit dem genannten Filter (122) und zum Zusammenziehen nach dem genannten Ausdehnen; und
b) wobei die genannte operative Verbindung eine operative Verbindung zwischen dem genannten Filter (122) und dem genannten wenigstens einen Ballon (130) während wenigstens eines Teils der genannten volumetrischen Ausdehnung des genannten wenigstens einen Ballons (130) umfasst.

3. Baugruppe (500)(600) nach Anspruch 2, wobei wenigstens ein Teil des genannten Filters (122) so konfiguriert ist, dass er mit einem luminalen Aspekt während des genannten Zusammenziehens des genannten wenigstens einen Ballons (130) entfernbar verbunden bleibt.

4. Baugruppe (500) (600) nach Anspruch 2, wobei wenigstens ein Teil des genannten Filters (122) einen Haftkleber mit einer Affinität für ein mit einem luminalen In-vivo-Aspekt assoziiertes Gewebe beinhaltet.

5. Baugruppe (500) (600) nach Anspruch 4, die eine Kompressionshülse (134) aufweist, die eine im Wesentlichen gekrümmte Wand mit einem proximalen Ende, einem distalen Ende und einem Lumen umfasst, das von dem genannten proximalen Ende zu dem genannten distalen Ende verläuft, wobei das genannte Lumen einen Querschnittsdurchmesser hat, der erheblich kleiner ist als der maximale Querschnittsdurchmesser des genannten luminalen Aspekts.

6. Baugruppe (500) (600) nach Anspruch 1, wobei das genannte Filter (122) einen distalen Abschnitt, einen proximalen Abschnitt, eine Öffnung zu dem genannten Filter (122), die mit dem genannten proximalen Abschnitt assoziiert ist, und wenigstens eine Strebe umfasst, die operativ mit dem genannten proximalen Abschitt assoziiert ist.

7. Baugruppe (500)(600) nach Anspruch 1, wobei das genannte Filter (122) Folgendes umfasst: a) einen distalen Abschnitt; b) einen proximalen Abschnitt; c) eine Öffnung zu dem genannten Filter, die mit dem genannten proximalen Abschnitt assoziiert ist; und d) wenigstens einen Schnurführungskanal (120), der wenigstens einen Abschnitt des genannten proximalen Abschnitts umfangsmäßig umgibt.

8. Baugruppe (500)(600) nach Anspruch 1, wobei das genannte Filter (122) ein Material mit Gewebebindungseigenschaften für einen mit einem In-vivo-Fluidstrom assoziierten Abschnitt von luminalem Gewebe umfasst.

9. Baugruppe (500) (600) nach Anspruch 1, mit einer Stenthaltespindel (232), die operativ mit dem genannten Stent (300)(200) verbunden ist, wenn der genannte Stent (300) (200) in einer kontaktierten Konfiguration ist, wobei die genannte Spindel (232) an einem distalen Abschnitt eines länglichen Katheters montiert ist.

10. Baugruppe (500)(600) nach einem der vorherigen Ansprüche, wobei der Stent (300)(200) eine metallische Basis beinhaltet und das Filter (122) aus einem synthetischen biostabilen Polymer oder einem natürlichen Polymer gebildet ist.

11. Baugruppe (500)(600) nach einem der vorherigen Ansprüche, wobei der Stent (300)(200) zum erheblichen Stützen der proximalen Öffnung (124) des Filters (122) beim Ausdehnen des Stents (300)(200) konfiguriert ist.

12. Baugruppe (500)(600) nach einem der vorherigen Ansprüche, wobei das Filter (122) zum Biegen nach außen konfiguriert ist, bis es durch einen luminalen Aspekt begrenzt wird.

## Revendications

1. Ensemble filtre (500)(600) pour recueillir les débris d'un vaisseau sténosé, comprenant :
(a) un dispositif vasculaire radialement expansible d'augmentation de l'écoulement comprenant un stent radialement déployable (300)(200) configuré pour ouvrir une lumière sténosée, ledit stent radialement déployable (300)(200) ayant une extrémité proximale, une extrémité distale et une lumière reliant lesdites extrémités proximales et distales ; et
(b) un filtre (122) comportant une ouverture proximale expansible (124) ayant une liaison coopérante avec ledit dispositif vasculaire radialement expansible d'augmentation de l'écoulement, ladite ouverture proximale (124) étant configurée pour être expansée conjointement avec l'expansion dudit dispositif, de sorte à ce que lorsque ladite ouverture (124) est en une configuration expansée, ledit filtre (122) est configuré pour filtrer des débris en provenance d'un écoulement de fluide dans lequel ledit filtre est disposé ;
dans lequel le filtre (122) est configuré pour être désengagé dudit dispositif expansible d'augmentation de l'écoulement et enlevé du vaisseau sténosé tandis que le stent radialement déployable (300)(200) demeure en place dans le vaisseau sténosé ;
**caractérisé en ce que** ledit dispositif vasculaire radialement expansible d'augmentation de l'écoulement comprend en outre :
une gaine externe (270) entourant sensiblement une surface externe dudit stent déployable (300)(200), ladite gaine externe (270) étant configurée pour s'expanser en association avec le déploiement dudit stent en cours de déploiement (300) (200) ; et
ladite liaison coopérante comprenant une liaison coopérante entre ledit filtre (122) et ladite gaine externe (270).

2. Ensemble (500)(600) selon la revendication 1, dans lequel ledit dispositif vasculaire radialement expansible d'augmentation de l'écoulement comprend :
a) au moins un ballonnet (130) configuré pour se déployer volumétriquement et, pendant au moins une partie dudit déploiement, se raccorder coopérativement audit filtre (122) et se contracter après ledit déploiement ; et
b) ladite liaison coopérante comprenant une liaison coopérante entre ledit filtre (122) et ledit au moins un ballonnet (130) pendant au moins une partie dudit déploiement volumétrique dudit au moins un ballonnet (130).

3. Ensemble (500)(600) selon la revendication 2, dans lequel au moins une partie dudit filtre (122) est configurée pour demeurer raccordée de façon amovible à un aspect luminal au cours de ladite contraction dudit au moins un ballonnet (130).

4. Ensemble (500)(600) selon la revendication 2, dans lequel au moins une partie dudit filtre (122) contient un adhésif sensible à la pression ayant une affinité pour un tissu associé avec un aspect luminal in vivo.

5. Ensemble (500)(600) selon la revendication 4, doté d'une gaine de compression (134) comprenant une paroi sensiblement incurvée ayant une extrémité proximale, une extrémité distale et une lumière s'étendant depuis ladite extrémité proximale jusqu'à ladite extrémité distale, ladite lumière ayant un diamètre en coupe transversale qui est sensiblement plus petit que le diamètre en coupe transversale maximal dudit aspect luminal.

6. Ensemble (500)(600) selon la revendication 1, dans lequel ledit filtre (122) comporte une partie distale, une partie proximale, une ouverture d'accès au filtre (122) associée à ladite partie proximale et au moins une armature associée de façon coopérante à ladite partie proximale.

7. Ensemble (500)(600) selon la revendication 1, dans lequel ledit filtre (122) comporte : a) une partie distale ; (b) une partie proximale ; c) une ouverture d'accès audit filtre associée à ladite partie proximale ; et d) au moins un conduit de guidage du fil (120) encerclant circonférentiellement au moins une partie de ladite partie proximale.

8. Ensemble (500)(600) selon la revendication 1, dans lequel ledit filtre (122) comprend un matériau présentant des propriétés de connexion aux tissus pour une partie du tissu luminal associé à un écoulement de fluide in vivo.

9. Ensemble (500)(600) selon la revendication 1, comportant une broche de retenue du stent (232) associée de façon coopérante audit stent (300)(200) lorsque ledit stent (300)(200) est en configuration de contact, ladite broche (232) étant montée sur une partie distale d'un cathéter allongé.

10. Ensemble (500)(600) selon l'une quelconque des revendications précédentes, dans lequel le stent (300)(200) est doté d'une base métallique et le filtre (122) est formé d'un polymère synthétique biostable ou d'un polymère naturel.

11. Ensemble (500)(600) selon l'une quelconque des revendications précédentes, dans lequel le stent (300)(200) est configuré pour soutenir sensiblement l'ouverture proximale (124) du filtre (122) au cours du déploiement du stent (300)(200).

12. Ensemble (500)(600) selon l'une quelconque des revendications précédentes, dans lequel le filtre (122) est configuré pour se fléchir vers l'extérieur jusqu'à ce qu'il soit limité par un aspect luminal.
